# EUROPEAN PATENT APPLICATION

(11) **EP 4 729 086 A1**
(43) Date of publication of application: **22.04.2026**
(21) Application number: 24207534.9
(22) Date of filing: 18.10.2024
(51) Int. Cl.: A61M 5/14, A61M 5/142

(54) **BUTTON MODULE AND PATCH TYPE INFUSION PUMP**

(71) Applicant: Syai UK Ltd, Cambridge CB2 8DL (GB)
(72) Inventor: Wang, Zhihua, Cambridge, CB2 8DL (GB)
(74) Representative: Nederlandsch Octrooibureau

(57) **Abstract**

The present application relates to the technical field of medical instruments, and disclosed thereby are a button module and a patch type infusion pump. The button module comprises an injection button and a button support. One side of a shell is provided with an installation slot, and an opening is provided at the bottom of the installation slot so that the installation slot is communicated with an interior of the shell. The button support is clamped to the installation slot. A side of the button support away from the shell is provided with an installation hole, and the injection button passes through the installation hole and the opening, and may move along an axis of the installation hole relative to the button support. A surface of the injection button is provided with a first damping ring and a second damping ring, and an inner wall of the installation hole is provided with a first buckle. When the button module is in its initial state, the first buckle is clamped between the first damping ring and the second damping ring. When the button module moves towards the interior of the shell to inject the injection assembly, the first damping ring is located on a side of the first buckle towards the shell. The above button module cannot only achieve manual injection, but also achieve the effect of injecting the indwelling needle into place at once.

## Description

### TECHNICAL FIELD

The present application relates to the technical field of medical instruments, and particularly to a button module and a patch type infusion pump.

### BACKGROUND

Before injecting medicament into a user's body, the patch type infusion pump needs to first inject the indwelling needle into the user's body, and then inject the medicament into the user's body by injecting into the indwelling needle. At present, some patch type infusion pumps in the market require the use of auxiliary large syringes for injection, which leads to users not only having to wear the infusion pump but also assembling the syringe when using these patch type infusion pumps, resulting in a cumbersome wearing process and a cost increase because of auxiliary syringes. Although some patch type infusion pumps employ manual injection, users may pause the injection due to pain, resulting in the inability to inject the indwelling needle at once and affecting the user experience.

### SUMMARY

The present application provides a button module and a patch type infusion pump, which cannot only achieve manual injection, cost reduction, but also achieve the effect of injecting the indwelling needle into place at once.

In a first aspect, the present application provides a button module, which is applied to a patch type infusion pump; the patch type infusion pump comprises a shell and an injection assembly located inside the shell, and the button module comprises an injection button and a button support;
one side of the shell is provided with an installation slot, and an opening is provided at the bottom of the installation slot so that the installation slot is communicated with an interior of the shell; the button support is clamped to the installation slot;
a side of the button support away from the shell is provided with an installation hole, and the injection button passes through the installation hole and the opening, and the injection button may move along an axis of the installation hole relative to the button support; wherein
a surface of the injection button is provided with a first damping ring and a second damping ring distributed along the circumference of the installation hole, and the first damping ring and the second damping ring are arranged along the axis of the installation hole; an inner wall of the installation hole is provided with a first buckle;
when the button module is in its initial state, the first buckle is clamped between the first damping ring and the second damping ring; when the button module moves towards an interior of the shell to inject the injection assembly, the first damping ring is located on a side of the first buckle towards the shell.

The button module provided by the present application is to install the injection button on the shell of the patch type infusion pump through the button support, and move the injection button towards the interior of the shell, thereby driving the injection assembly to inject the indwelling needle into a user's body. The surface of the injection button is provided with a first damping ring and a second damping ring, and a first buckle is provided in the installation hole of the button support; when the injection button is in the initial state, the first buckle is clamped between the first damping ring and the second damping ring, thus the injection button is located in one end of the shell and in contact with the injection assembly. When a user manually presses the injection button, under the obstructing effect of the first buckle, the injection button moves at least the size of the first buckle along the axis of the installation hole towards the interior of the shell, so that the first damping ring passes over the first buckle, in order to achieve effective movement of the injection button. During this process, the injection button drives the injection assembly to inject the indwelling needle into a user's body. Therefore, the button module in the present application may be designed according to the size of the first buckle along the axis of the installation hole, so that the user may inject the indwelling needle into place at once when pressing the injection button, improving the user experience. Therefore, the button module in the present application cannot only achieve manual injection, but also achieve the effect of injecting the indwelling needle into place at once.

In some possible embodiments, there are multiple first buckles provided, and the multiple first buckles are uniformly distributed at intervals around the circumference of the installation hole.

In some possible embodiments, the side of the first buckle away from the inner wall of the installation hole is provided with a guide surface, which is used to guide the injection button moving towards the interior of the shell.

In some possible embodiments, the first damping ring comprises multiple first annular projection portions, which are distributed at intervals around the circumference of the installation hole, and each of the first annular projection portions is arranged in one-to-one correspondence with each of the first buckles; the second damping ring comprises multiple second annular projection portions, which are arranged in one-to-one correspondence with the first annular projection portions.

In some possible embodiments, the inner wall of the installation hole is further provided with multiple second buckles, and the multiple second buckles are uniformly distributed around the circumference of the installation hole;
the second buckle is located on the side of the first buckle away from the shell, and each of the second buckles abuts against the injection button.

In some possible embodiments, the side of the button support towards the button support is provided with multiple third buckles, and the shell is provided with clamping holes which are in one-to-one correspondence with the multiple third buckles; each of the third buckles is buckled to the corresponding clamping hole.

In some possible embodiments, the button support is detachably connected to the shell, and the button module further comprises a sealing cover plate, which is connected to the shell and may slide relative to the shell, so that the sealing cover plate covers a notch of the installation slot.

In some possible embodiments, a fool-proofing spring plate is provided inside the shell, and the fool-proofing spring plate is used to fix the sealing cover plate and the shell relative to each other when the sealing cover plate covers the installation slot. In some possible embodiments, a side of the sealing cover plate towards the installation slot is provided with a silicone pad, and the silicone pad abuts against a bottom of the installation slot;
the silicone pad is of an annular structure, and the silicone pad is arranged around the circumference of the opening when the sealing cover plate covers the installation slot.

In a second aspect, the present application provides a patch type infusion pump, comprising a shell, an injection assembly and the button module in any possible embodiment of the first aspect, wherein the injection assembly is located inside the shell, and the button module is used to drive the injection assembly to inject an indwelling needle into a user's body.

### BRIEF DESCRIPTION OF DRAWINGS

FIG. 1 is a schematic diagram of the explosive structure of the patch type infusion pump in the embodiment of the present application;
FIG. 2 is a partial structural sectional view of the button module in the embodiment of the present application;
FIG. 3 is a sectional view of the patch type infusion pump in the initial state in the embodiment of the present application;
FIG. 4 is a structural diagram of the button support in the embodiment of the present application;
FIG. 5 is a structural diagram of the injection button in the embodiment of the present application;
FIG. 6 is a structural diagram of the button support from another perspective in the embodiment of the present application;
FIG. 7 is a structural diagram of the patch type infusion pump in the embodiment of the present application when the button module is removed;
FIG. 8 is a structural diagram of the installation slot covered by the sealing cover plate in FIG. 7.
FIG. 9 is a sectional view of the sealing cover plate and the front shell in the embodiment of the present application;
FIG. 10 is a sectional view of the sealing cover plate of the patch type infusion pump in the first station in the embodiment of the present application; and
FIG. 11 is a sectional view of the sealing cover plate in the second station in FIG. 10.

In the drawings:
100-shell; 110-front shell; 111-installation slot; 1111-opening; 120-rear shell; 200-button module; 210-button support; 211-installation portion; 2111-first installation plate; 2112-second installation plate; 212-installation hole; 2121-first buckle; 21211-guide surface; 2122-second buckle; 213-third buckle; 220-injection button; 221-first damping ring; 2211-first annular projection portion; 222-second damping ring; 2221-second annular projection portion; 230-sealing cover plate; 231-limit projection; 240-silicone pad; 300-injection assembly; 310-injection support; 320-catheter; 400-fool-proofing spring plate.

### DETAILED DESCRIPTION OF THE EMBODIMENTS

Technical solutions in the embodiments of the present invention will be described clearly and completely in combination with figures in the embodiments of the invention. Obviously, the described embodiments are only part, but not all, of the embodiments of the invention. All other embodiments obtained by those of ordinary skill in the art without creative work based on the embodiments of the present invention are within the scope of protection of the present invention.

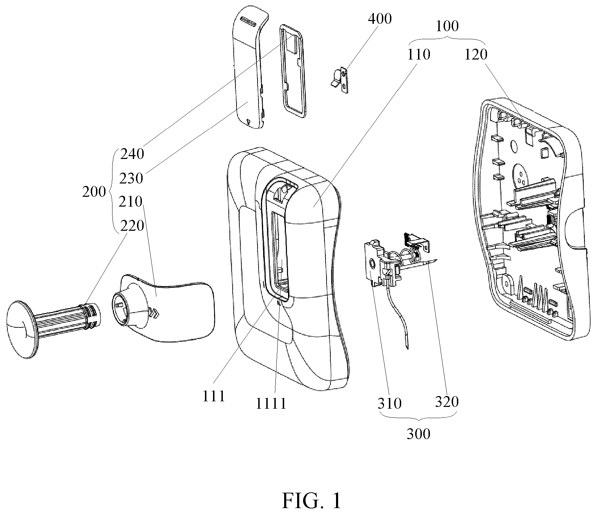

Referring to FIG. 1, the patch type infusion pump in the embodiment of the present application may comprise a shell 100, a button module 200, and an injection assembly 300, wherein the shell 100 may comprise a front shell 110 and a rear shell 120, which may be buckled with each other, so that the interior of the shell 100 is provided with a storage space for storing the injection assembly 300. The button module 200 is located on the front shell 110, and may be used to provide driving force to the injection assembly 300 inside the shell 100, so that the injection assembly 300 may inject the indwelling needle into a user's body.

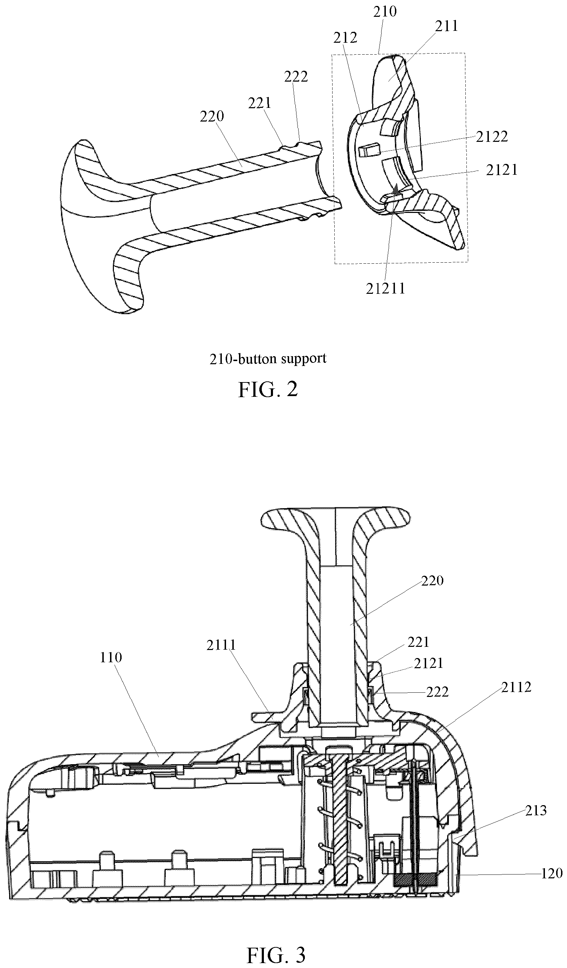

As shown in FIG. 1, the button module 200 comprises a button support 210, an injection button 220, and a sealing cover plate 230, wherein the button support 210 and the sealing cover plate 230 are both connected to the front shell 110, and the injection button 220 is installed on the button support 210. Specifically, referring to both FIG. 1 and FIG. 2, the button support 210 comprises an installation portion 211 and an installation hole 212. The side of the front shell 110 away from the rear shell 120 is provided with an installation slot 111 that matches the shape of the installation portion 211, so that the installation portion 211 may be accommodated in the installation slot 111. In addition, each side of the installation portion 211 may abut against the inner wall of the installation slot 111, so that the button support 210 may maintain a good positioning effect with the installation slot 111.

The installation hole 212 is connected to the side of the installation portion 211 away from the installation slot 111. The axis of the installation hole 212 is consistent with the injection direction of the injection assembly 300. The bottom of the installation slot 111 is provided with an opening 1111, which may be used to connect the installation slot 111 with the interior of the shell 100. The installation hole 212 is directly opposite the opening 1111. One end of the injection button 220 passes through the installation hole 212 and the opening 1111 and extends into the interior of the shell 100. The injection assembly 300 may comprise an injection support 310 and a catheter 320. The indwelling needle is located inside the catheter 320, which is connected to the injection support 310. The needle injection button 220 may be in contact with the needle injection bracket 310. The injection button 220 may move relative to the installation hole 212 along the axis of the installation hole 212 towards the interior of the shell 100, in order to drive the injection support 310 to move, so that the injection support 310 may drive the indwelling needle in the catheter 320 into a user's body.

Referring to FIGS. 2 and 3, the surface of the injection button 220 passing through the installation hole 212 is provided with a first damping ring 221 and a second damping ring 222. The first damping ring 221 is located on the side of the second damping ring 222 away from the shell 100, and both the first damping ring 221 and the second damping ring 222 are arranged around the circumference of the installation hole 212. The first damping ring 221 and the second damping ring 222 are arranged at intervals along the axis of the installation hole 212. The inner wall of the installation hole 212 is provided with a first buckle 2121. When the injection button 220 is in the initial state, the first buckle 2121 is clamped between the first damping ring 221 and the second damping ring 222. At this time, the injection button 220 and the button support 210 remain relatively fixed without external force under the cooperation of the first buckle 2121, the first damping ring 221, and the second damping ring 222. When the user manually presses the injection button 220, a large driving force may be applied to the injection button 220, which may drive the injection button 220 to move towards the interior of the shell 100, thereby causing the first damping ring 221 to move to the side of the first buckle 2121 towards the shell 100. It should be understood that during this process, when the injection button 220 moves, the injection assembly 300 is driven to inject. Since in the initial state, the first buckle 2121 is clamped between the first damping ring 221 and the second damping ring 222, in order to ensure that the injection button 220 may move relative to the button support 210, the injection button 220 needs to move at least the size of the first buckle 2121 along the axis of the installation hole 212 at once, in order to achieve effective pressing and injection of the injection button 220. In this way, by designing the size of the first buckle 2121 along the axis of the installation hole 212, the injection may be completed after the first damping ring 221 passes over the first buckle 2121, achieving one-time injection on the basis of manual injection.

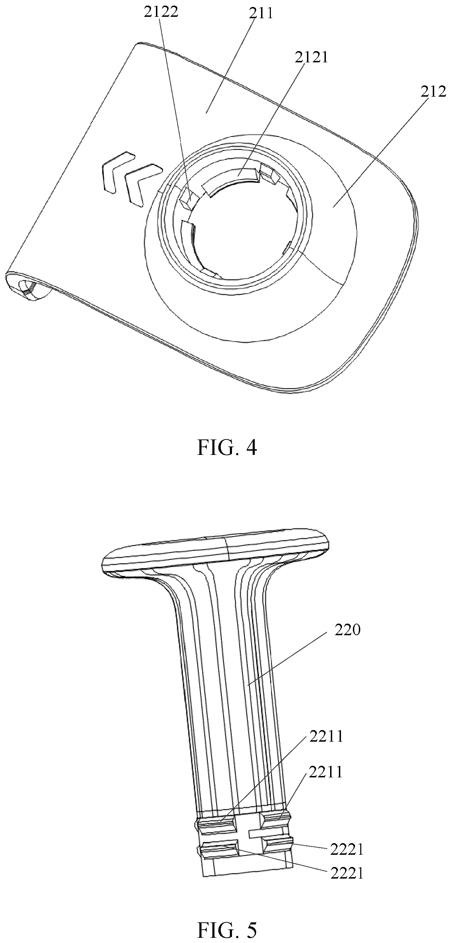

In some embodiments, referring to FIGS. 4 and 5, there are multiple first buckles 2121, which are uniformly distributed at intervals around the axis of the installation hole 212. This ensures that the injection button 220 and the button support 210 are balanced in force, ensuring that the injection support 310 may move along the axis of the installation hole 212. Based on this, the first damping ring 221 may comprise multiple first annular projection portions 2211 arranged at intervals, and the second damping ring 222 may comprise multiple second annular projection portions 2221 arranged at intervals, wherein the first buckle 2121, the first annular projection portions 2211, and the second annular projection portions 2221 are arranged in one-to-one correspondence, and the first buckle 2121, the first annular projection portions 2211, and the second annular projection portions 2221 may also be made to have the same arc size along the circumference of the installation hole 212 to achieve a better damping effect.

Furthermore, referring to FIG. 2 again, the side of the first buckle 2121 away from the inner wall of the installation hole 212 is provided with a guide surface 21211, which may be used to guide the injection button 220 moving towards the interior of the shell 100. In specific implementation, as shown in FIG. 2, there is a certain included angle between the guide surface 21211 and the inner wall of the installation hole 212, which ranges from 0 to 90 degrees, so that the guide surface 21211 is inclined relative to the inner wall of the installation hole 212. Moreover, the end of the guide surface 21211 near the inner wall of the installation hole 212 is higher than the end away from the inner wall of the installation hole 212 in the axis direction of the installation hole 212. When the injection button 220 moves towards the interior of the shell 100, the guide surface 21211 may guide the first damping ring 221, making it easier for the first damping ring 221 to pass over the first buckle 2121.

In addition, as an alternative embodiment, referring to FIG. 5, the cross-section of the first annular projection portion 2211 may be of a conical structure. When the first buckle 2121 is clamped between the first annular projection portion 2211 and the second annular projection portion 2221, the lower surface of the first annular projection portion 2211 may be as close as possible to the guide surface 21211 of the first buckle 2121 to improve the clamping effect therebetween. Similarly, the cross-section of the second annular projection portion 2221 may also be of a conical structure. When assembling the injection button 220 and the button support 210 from top to bottom, the second annular projection portion 2221 may more easily pass over the first buckle 2121 and clamp it between the first damping ring 221 and the second damping ring 222.

It is worth noting that when the first damping ring 221 and the second damping ring 222 are designed as segmented structures, it not only facilitates the control of the resistance values of the first damping ring 221 and the second damping ring 222, but also facilitates production. Similarly, the segmented design of multiple first buckles 2121 may also facilitate the control of the resistance value of the first buckles 2121 and facilitate production.

In some embodiments, referring to FIG. 2 or FIG. 4, multiple second buckles 2122 may be provided at the inner wall of the installation hole 212 at one side where the first buckle 2121 departs from the shell 100, and these second buckles 2122 are uniformly distributed around the circumference of the installation hole 212. When the injection button 220 passes through the installation hole 212, the second buckle 2122 is located on the side of the first damping ring 221 away from the first buckle 2121. Each second buckle 2122 may abut against the surface of the injection button 220, so that multiple second buckles 2122 cooperate to limit the injection button 220 in the circumference of the installation hole 212, preventing the injection button 220 from tilting and allowing the injection button 220 to only move along the axis of the installation hole 212. In addition, the number of second buckles 2122 is the same as the number of first buckles 2121, and each second buckle 2122 may be directly opposite the gap between adjacent first buckles 2121. When assembling the injection button 220 from top to bottom with the button support 210, the first annular projection portion 2211 and the second annular projection portion 2221 may be kept away from the second buckle 2122 and clamped to the first buckle 2121, simplifying the assembly of the injection button 220 and the button support 210 and saving time and effort.

In addition, in this embodiment, the design of the first damping ring 221, the second damping ring 222, the first buckle 2121, and the second buckle 2122 may also limit the injection button 220, so that the injection button 220 may be coaxial with the injection support 310, thereby avoiding central position offset between the two during transportation, which may affect the injection effect.

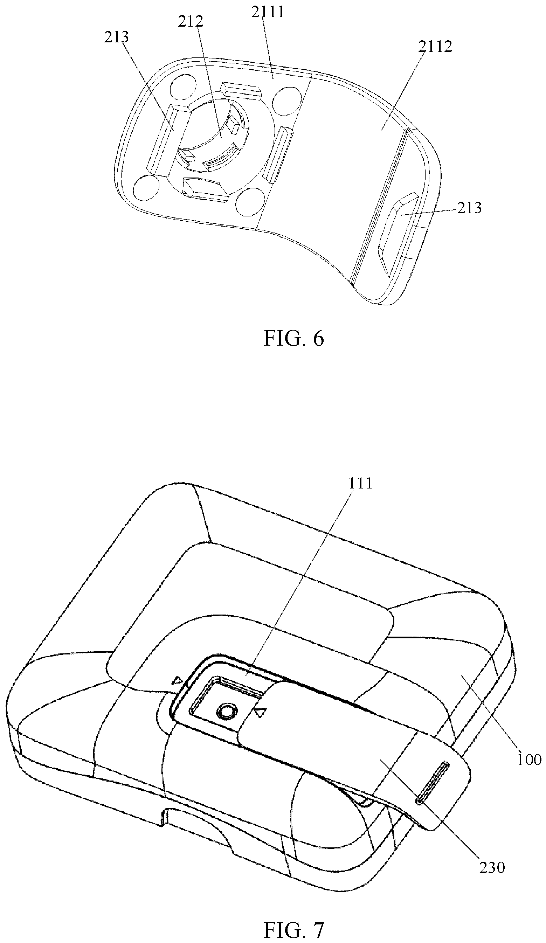

Referring to FIGS. 3 and 6 together, the installation portion 211 is roughly of an L-shaped structure, with the same bending angle as the end of the front shell 110, so that when a portion of the installation portion 211 is clamped to the installation slot 111, the portion installed outside the installation slot 111 may be attached to the side wall surface of the front shell 110. Specifically, the installation portion 211 may be regarded as comprising a first installation plate 2111 and a second installation plate 2112. The first installation plate 2111 and the second installation plate 2112 may be designed as a single unit, with the first installation plate 2111 directly opposite the large surface of the front shell 110 and the second installation plate 2112 directly opposite the side wall of the front shell 110.

The side of the installation portion 211 towards the shell 100 is provided with multiple third buckles, wherein at least one third buckle 213 is arranged on the second installation plate 2112, and the rest third buckles 213 are arranged on the first installation plate 2111 around the installation hole 212. As an alternative embodiment, a portion of the second installation plate 2112 may be directly opposite the side wall of the rear shell 120. At this time, the third buckle 213 provided on the second installation plate 2112 is located at the position where the second installation plate 2112 is directly opposite the side wall of the rear shell 120. The rear shell 120 is provided with clamping holes (not shown in the figure) in one-to-one correspondence with the third buckle 213 on the second installation plate 2112, so that the third buckle 213 on the second mounting plate 2112 may be buckled into the clamping hole of the rear shell 120, thereby ensuring that the button support 210 is relatively fixed with the shell 100 in the height direction of the shell 100. In addition, the front shell 110 is provided with clamping holes (not shown in the figure) in one-to-one correspondence with the third buckles 213 on the first installation plate 2111, so that the third buckles 213 on the first installation plate 2111 may be clamped into the clamping hole of the front shell 110, thereby ensuring the relative fixation between the button support 210 and the shell 100, and ensuring the fixed position of the injection button 220 during use.

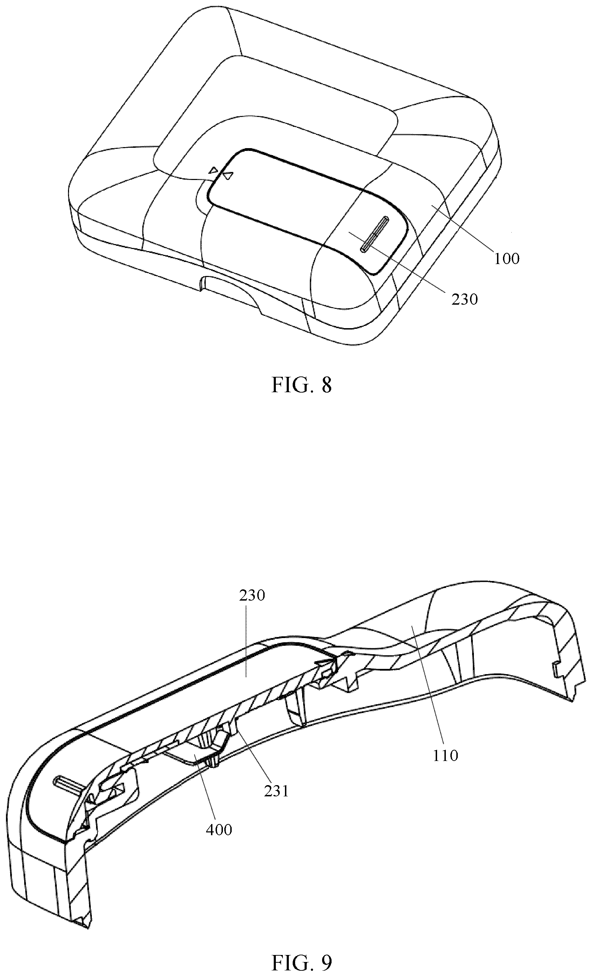

It should be understood that in this embodiment, the button support 210 is installed on the shell 100 by buckling the buckle and the clamping hole, which may also facilitate the removal of the button support 210 from the shell 100 after the injection is completed. That is to say, the button support 210 in this embodiment is detachably connected to the shell 100. Specifically, when removing the button support 210, the second installation plate 2112 may be lifted relative to the shell 100 first, so that the third buckle 213 on the second installation plate 2112 is disengaged from the corresponding clamping hole. Then the injection button 220 is lifted. At this point, the first damping ring 221 of the injection button 220 is located below the first buckle 2121. When lifting the injection button 220, the first damping ring 221 contacts the bottom of the first buckle 2121, but the first damping ring 221 cannot pass over the first buckle 2121 and return to the state where the first damping ring 221 is located above the first buckle 2121. Therefore, at this point, the injection button 220 and the button support 210 are relatively fixed. Continuing to lift the injection button 220 may enable the third buckle 213 on the first installation plate 2111 to disengage from the corresponding clamping hole, thereby removing the injection button 220 and the button support 210 as a whole from the shell 100. Referring to FIGS. 7 and 8, the sealing cover plate 230 is connected to the front shell 110. When the button support 210 is removed from the shell 100, the sealing cover plate 230 may slide relative to the front shell 110, thereby covering the notch of the installation slot 111, making the interior of the shell 100 in a sealed state to ensure the subsequent injection effect. For example, in this embodiment, the opposite sides of the sealing cover plate 230 may be connected to the side walls of the installation slot 111, and the sealing cover plate 230 may be slidably connected to the side walls of the installation slot 111 through a sliding block structure to achieve relative sliding between the sealing cover plate 230 and the installation slot 111. In addition, the sealing cover plate 230 may also be connected to the shell 100 through a spring. When the button support 210 is installed on the shell 100, the sealing cover plate 230 is in the first position, and the button support 210 abuts against the sealing cover plate 230. At this time, the spring is in a compressed state, causing the sealing cover plate 230 to have a tendency to slide relative to the shell 100. After the button support 210 is removed, the elastic force of the spring is released to drive the sealing cover plate 230 to slide to the second station, thereby causing the sealing cover plate 230 to cover the installation slot 111.

As an alternative embodiment, when the sealing cover plate 230 covers the installation slot 111, the spring is still in a compressed state, which may make the sealing cover plate 230 have a tendency to further slide forwards relative to the shell 100, thereby improving the sealing effect between the sealing cover plate 230 and the installation slot 111.

Of course, in some other embodiments, the sealing cover plate 230 is slidably connected to the side wall of the installation slot 111 through a sliding block structure. After removing the button support 210, the user may also manually push the sealing cover plate 230 to slide, so that the sealing cover plate 230 covers the installation slot 111.

Based on this, referring to FIGS. 9 to 11, the side of the sealing cover plate 230 towards the shell 100 is also provided with a limiting protrusion 231. The interior of the shell 100 is provided with an error-proofing spring plate 400. When the sealing cover plate 230 is in the first station, the fool-proofing spring plate 400 is located in front of the limit projection 231. When the sealing cover plate 230 is in the second station, the fool-proofing spring plate 400 is located behind the limit projection 231. It should be noted that the sliding direction of the sealing cover plate 230 is used as a reference for the front and behind. The end of the limit projection 231 near the shell 100 for installing the button support 210 is bent towards the sealing cover plate 230 at a certain angle. When the sealing cover plate 230 slides from the first station to the second station, the limit projection 231 may squeeze the fool-proofing spring plate 400, causing deformation of the fool-proofing spring plate 400 and facilitating the limit projection 231 to pass over the fool-proofing spring plate 400. After the limit projection 231 leaves, the fool-proofing spring plate 400 releases its elastic force, returns to its initial state, and comes into contact with the surface of the limit projection 231, thereby restricting the sliding of the sealing cover plate 230 from the second station towards the first station, ensuring the sealing effect of the sealing cover plate 230.

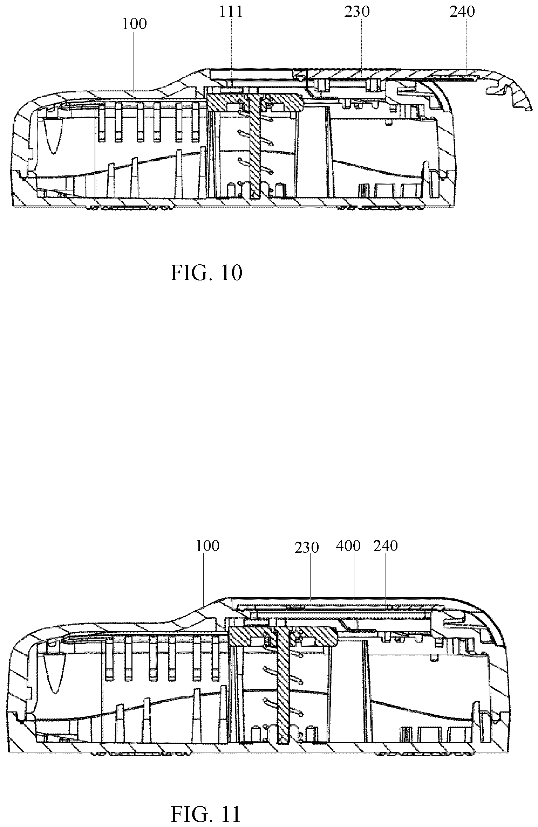

Furthermore, as shown in FIG. 1, the side of the sealing cover plate 230 towards the shell 100 is also provided with a silicone pad 240, which is of an annular structure, and fixed on the sealing cover plate 230. As an alternative embodiment, the sealing cover plate 230 and the silicone pad 240 are injection molded one-piece structure, ensuring that the silicone pad 240 will not fall off from the sealing cover plate 230 or deform during the sliding process of the sealing cover plate 230 relative to the shell 100. Referring to FIGS. 10 and 11 again, when the sealing cover plate 230 is in the second position, the silicone pad 240 is arranged around the opening 1111 at the bottom of the installation slot 111. At this time, the side surface of the silicone pad 240 away from the sealing cover plate 230 abuts against the bottom of the installation slot 111, so that an interference sealing is formed between the sealing cover plate 230 and the shell 100 by squeezing the silicone pad 240, further ensuring the sealing effect. At the same time, after the sealing cover plate 230 covers the installation slot 111, the shell 100 may also have a complete shape, which is conducive to improving the aesthetics of the product.

It will be apparent to those skilled in the art that various amendments and variations may be made to the embodiment of the present invention without departing from the spirit and scope of the present invention. In this way, if these amendments and variations of the present invention are within the ranges of the claims of the present invention and equivalent technologies thereof, the present invention has also intended to contain those amendments and modifications.

## Claims

1. A button module applied to a patch type infusion pump, the patch type infusion pump comprising a shell and an injection assembly located inside the shell, wherein the button module comprises an injection button and a button support;
wherein one side of the shell is provided with an installation slot, and an opening is provided at the bottom of the installation slot so that the installation slot is communicated with the interior of the shell; and the button support is clamped to the installation slot;
wherein the side of the button support away from the shell is provided with an installation hole, and the injection button passes through the installation hole and the opening, and the injection button is configured to move along an axis of the installation hole relative to the button support; wherein
wherein a surface of the injection button is provided with a first damping ring and a second damping ring distributed along a circumference of the installation hole, and the first damping ring and the second damping ring are arranged along the axis of the installation hole; an inner wall of the installation hole is provided with a first buckle;
wherein when the button module is in its initial state, the first buckle is clamped between the first damping ring and the second damping ring; when the button module moves towards an interior of the shell to inject the injection assembly, the first damping ring is located on a side of the first buckle towards the shell.

2. The button module according to claim 1, wherein there are multiple first buckles provided, and the multiple first buckles are uniformly distributed at intervals around the circumference of the installation hole.

3. The button module according to claim 2, wherein the side of the first buckle away from the inner wall of the installation hole is provided with a guide surface, which is used to guide the injection button moving towards an interior of the shell.

4. The button module according to claim 2, wherein the first damping ring comprises multiple first annular projection portions, which are distributed at intervals around the circumference of the installation hole, and each of the first annular projection portions is arranged in one-to-one correspondence with each of the first buckles;
wherein the second damping ring comprises multiple second annular projection portions, which are arranged in one-to-one correspondence with the first annular projection portions.

5. The button module according to claim 1, wherein the inner wall of the installation hole is further provided with multiple second buckles, and the multiple second buckles are uniformly distributed around the circumference of the installation hole; wherein the second buckle is located on a side of the first buckle away from the shell, and each of the second buckles abuts against the injection button.

6. The button module according to claim 1, wherein the side of the button support towards the button support is provided with multiple third buckles, and the shell is provided with clamping holes which are in one-to-one correspondence with the multiple third buckles; each of the third buckles is buckled to the corresponding clamping hole.

7. The button module according to claim 1, wherein the button support is detachably connected to the shell, and the button module further comprises a sealing cover plate, which is connected to the shell and is configured to slide relative to the shell, so that the sealing cover plate covers a notch of the installation slot.

8. The button module according to claim 7, wherein a fool-proofing spring plate is provided inside the shell, and the fool-proofing spring plate is used to fix the sealing cover plate and the shell relative to each other when the sealing cover plate covers the installation slot.

9. The button module according to claim 7, wherein a side of the sealing cover plate towards the installation slot is provided with a silicone pad, and the silicone pad abuts against a bottom of the installation slot;
wherein the silicone pad is of an annular structure, and the silicone pad is arranged around a circumference of the opening when the sealing cover plate covers the installation slot.

10. A patch type infusion pump, comprising a shell, an injection assembly and the button module according to any of claims 1-9, wherein the injection assembly is located inside the shell, and the button module is used to drive the injection assembly to inject an indwelling needle into a user's body.
